(19) Europäisches Patentamt
European Patent Office
Office européen des brevets

(11) **EP 4 070 734 B1**

(12) **EUROPEAN PATENT SPECIFICATION**

(45) Date of publication and mention
of the grant of the patent:
**05.08.2026  Bulletin 2026/32**

(21) Application number: **20897575.5**

(22) Date of filing: **04.12.2020**

(51) International Patent Classification (IPC):
***A61B 5/0507*** *(2021.01)*        ***A61B 90/00*** *(2016.01)*
***A61B 10/00*** *(2006.01)*        ***A61B 5/00*** *(2006.01)*

(52) Cooperative Patent Classification (CPC):
**A61B 90/39; A61B 5/0507; A61B 5/4312;**
**A61B 10/00;** A61B 2090/3908; A61B 2090/3937;
A61B 2090/397; A61B 2090/3991

(86) International application number:
**PCT/JP2020/045239**

(87) International publication number:
**WO 2021/112222 (10.06.2021 Gazette 2021/23)**

(54) **SUPPLEMENTARY PATCH AND TESTING PATCH SET**

ZUSÄTZLICHES PATCH UND PRÜFPATCH-SET

PATCH AUXILIAIRE ET ENSEMBLE PATCH DE TEST

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **06.12.2019  JP 2019221550**

(43) Date of publication of application:
**12.10.2022  Bulletin 2022/41**

(73) Proprietor: **TOPPAN INC.**
**Tokyo 110-0016 (JP)**

(72) Inventors:
• **NOMURA Saeko**
**Tokyo 110-0016 (JP)**
• **TANABE Junya**
**Tokyo 110-0016 (JP)**
• **TODE Ryohei**
**Tokyo 110-0016 (JP)**
• **YAMAZAKI Tsukasa**
**Tokyo 110-0016 (JP)**

(74) Representative: **TBK**
**Bavariaring 4-6**
**80336 München (DE)**

(56) References cited:
WO-A1-2006/052951        WO-A1-2012/048020
WO-A1-2017/057524        WO-A1-2020/059861
WO-A1-2020/145385        JP-A- 2004 347 660
JP-A- 2006 130 865        JP-A- 2019 510 593
KR-A- 20150 082 793      US-A- 5 662 110
US-A1- 2011 223 418

**Description**

[Technical Field]

The present invention relates to the use of an adhesive supplementary marker, together with an adhesive examination marker and an adhesive examination marker set including the adhesive supplementary marker and the adhesive examination marker.

[Background Art]

Mammography using microwaves has been proposed as an examination method for breast cancer (see, for example, PTL 1). In mammography using microwaves, the subject does not feel pain during an examination because it is not necessary to compress the breast of the subject to be examined. Further, the subject is not exposed to ionizing radiation since X-rays are not used in mammography using microwaves.

WO 2006/052951 A describes an ink jet image recording medium comprising an adhesive layer having a thickness of 20 to 1000 $\mu$m, which contains 50 to 95% by weight of a hot melt pressure-sensitive adhesive and 5 to 50% by weight of a film forming component; and an image receiving layer having a thickness of 15 to 40 $\mu$m provided on the adhesive layer.

JP 2004 347660 A describes a method for rubbing even a lens to which oil repellent coating is applied without causing axial deviation, wherein an adhesive tape is stuck to both sides of the lens to be worked before setting the lens in a lens rubbing machine. The tape consists of a tape with a pressure sensitive adhesive obtained by coating the surface of a polymer film which has such transparency that light transmittance is $\geq$80%, whose thickness is 10 to 100$\mu$m and whose Young's modulus is $\geq$1Gpa with a pressure sensitive adhesive, and is stuck by pressing the pressure sensitive adhesive side to the surface of the lens.

US 2011/223418 A1 describes a method for treating an optical lens coated on at least one of the main surfaces thereof with a hydrophobic and/or oleophobic external coating comprising a step of depositing onto at least one part of the hydrophobic and/or oleophobic external coating a temporary adhesive composite film, said adhesive composite film comprising a pre-formed film, one main surface of which is coated with a pressure-sensitive adhesive layer, said adhesive layer directly contacting the hydrophobic and/or oleophobic coating, the pre-formed film modulus of elasticity in tension E' being higher than or equal to 4200 MPa, and the rupture stress of the assembly composed of said adhesive film bonded to a polycarbonate specimen coated with a fluorinated silane layer is higher than or equal to 0.05 MPa.

WO 2012/048020 A1 describes an apparatus and a method for imaging a tissue. The method includes transmitting a first microwave frequency signal to and receiving a first total signal from the tissue at a first position. A second microwave frequency signal is transmitted to and a second total signal received from the tissue at a second position.

[Citation List]

[Patent Literature]

PTL 1: WO 2017/057524

[Summary of Invention]

[Technical Problem]

In microwave mammography, three-dimensional images are generated by scanning the breast with a probe. Then, the three-dimensional images thus generated are used to diagnose whether the subject suffers from breast cancer. The breast as a target of the scan has a level difference between the breast body and the nipple portion, and hence the scan of the breast with the probe may be obstructed by the level difference.

Note that such a problem occurs not only in the case where the examination target is the breast, but also in cases where the examination target is another part of a human body, and this examination target has a level difference due to a protruding portion located on a surface of the examination target.

The present invention has been made to provide an adhesive supplementary marker and an adhesive examination marker set that enable scans with a probe to be performed more smoothly.

[Solution to Problem]

In order to solve the above problem, there is provided a use of an adhesive supplementary marker together with an adhesive examination marker to be attached to an examination target of image diagnosis using microwaves, the adhesive supplementary marker is disposed between the adhesive examination marker and the examination target and pressed toward the examination target by the adhesive examination marker in a state in which the adhesive examination marker is attached to the examination target, the adhesive supplementary marker including a laminate including a base film having a thickness of 20 $\mu$m or less, and a pressure-sensitive adhesive layer configured to be attached to the examination target, the base film having a Young's modulus of 1.0 GPa or more.

In order to solve the above problem, there is further provided an adhesive examination marker set as defined in claim 7 including: the adhesive supplementary marker; and an adhesive examination marker configured to be attached to the examination target so that the adhesive examination marker covers the adhesive supplementary marker attached to the examination target.

According to the above configurations, in a state in which the laminate is attached to the examination target so that the laminate covers a protruding portion of the examination target, the Young's modulus of the base film enables the laminate to adhere to the examination target while pressing the protruding portion in a direction in which the height of the protruding portion is reduced. When such an adhesive supplementary marker is applied to the examination target, a level difference of the examination target is reduced by the adhesive supplementary marker. Thus, scans with a probe can be performed more smoothly.

[Brief Description of Drawings]

Fig. 1 is a cross-sectional view of a structure of an adhesive supplementary marker according to an embodiment.

Fig. 2 is a plan view illustrating the configuration of the adhesive supplementary marker illustrated in Fig. 1.

Fig. 3 is a cross-sectional view of a structure of an adhesive examination marker unit to be used together with the adhesive supplementary marker illustrated in Fig. 1.

Fig. 4 is a plan view of a structure of the adhesive examination marker unit illustrated in Fig. 3.

Fig. 5 is a schematic diagram illustrating the usage of an adhesive examination marker set including the adhesive supplementary marker illustrated in Fig. 1 and the adhesive examination marker unit illustrated in Fig. 3.

Fig. 6 is another schematic diagram illustrating the usage of the adhesive examination marker set including the adhesive supplementary marker illustrated in Fig. 1 and the adhesive examination marker unit illustrated in Fig. 3.

Fig. 7 is still another schematic diagram illustrating the usage of the adhesive examination marker set including the adhesive supplementary marker illustrated in Fig. 1 and the adhesive examination marker unit illustrated in Fig. 3.

Fig. 8 is a table showing evaluation results of Examples and Comparative Examples of the adhesive supplementary marker.

[Description of Embodiments]

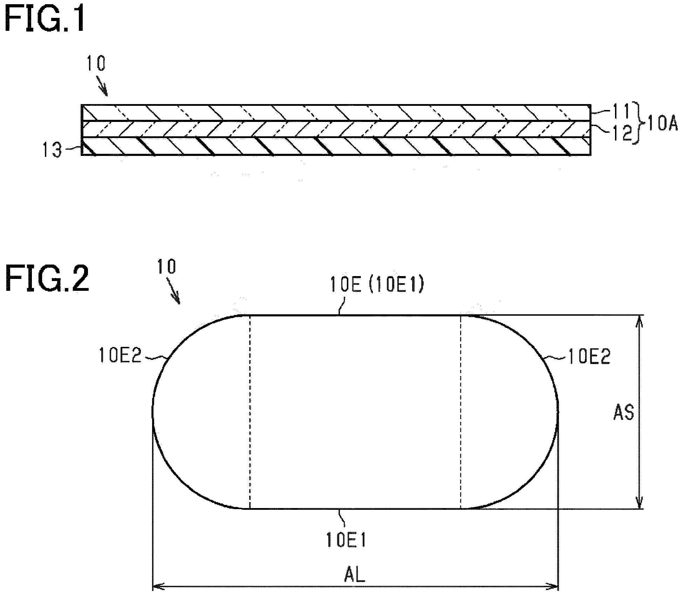

With reference to Figs. 1 to 8, an adhesive supplementary marker according to an embodiment will be described. The adhesive supplementary marker, an adhesive examination marker unit to be used together with the adhesive supplementary marker, usage of the adhesive supplementary marker and the adhesive examination marker unit, and Examples will be described in this order.

[Adhesive Supplementary Marker]

With reference to Figs. 1 and 2, the adhesive supplementary marker will be described. Fig. 1 illustrates a cross-sectional structure of the adhesive supplementary marker along a crosssection orthogonal to a plane in which the adhesive supplementary marker extends. Fig. 2 illustrates a planar configuration of the adhesive supplementary marker as viewed from a viewpoint facing the plane in which the adhesive supplementary marker extends.

The adhesive supplementary marker is used together with an adhesive examination marker to be attached to an examination target of image diagnosis using microwaves. The adhesive supplementary marker constitutes an adhesive examination marker set cooperatively with the adhesive examination marker. During use of the adhesive supplementary marker, the adhesive supplementary marker is disposed between the adhesive examination marker and the examination target, and pressed toward the examination target by the adhesive examination marker. In this embodiment, the examination target is a breast, and the adhesive examination marker and the adhesive supplementary marker are adhesive markers to be used in mammography using microwaves. In this embodiment, it is particularly preferred to use microwaves in a wavelength band of 1 GHz or more and 10 GHz or less.

As illustrated in Fig. 1, an adhesive supplementary marker 10 includes a base film 11 and a pressure-sensitive adhesive layer 12. The pressure-sensitive adhesive layer 12 is laminated on the base film 11 and attached to the breast. A laminate of the base film 11 and the pressure-sensitive adhesive layer 12 constitutes an adhesive supplementary marker body 10A. The adhesive supplementary marker body 10A satisfies the following conditions 1-1 and 1-2.

(Condition 1-1) The base film 11 has a thickness of 20 $\mu$m or less.
(Condition 1-2) The base film 11 has a Young's modulus of 1 GPa or more.

In a state in which the adhesive supplementary marker body 10A is attached to the breast so that the adhesive supplementary marker body 10A covers the nipple portion of the breast, the Young's modulus of the base film 11 enables the adhesive supplementary marker body 10A to adhere to the breast while pressing the nipple portion in a direction in which the height of the nipple portion is reduced. When such an adhesive supplementary marker 10 is applied to the breast, a level difference of the breast is reduced by the adhesive supplementary marker 10. Thus, scans with a probe can be performed more smoothly.

The Young's modulus is calculated according to "Plastics - Determination of tensile properties - Part 3: Test conditions for films and sheets" of JIS K 7127:1999 (ISO 527-3:1995), and "Plastics - Determination of tensile properties - Part 1: General principles" of JIS K 7161-1:2014 (ISO 527-1:2012). The Young's modulus is defined in 3.9 of JIS K 7161-1:2014. Note that the Young's modulus is calculated by the following equation (1-1) specified in 10.3.2 "Chord slope" in 10 "Calculation and expression of results" of this standard. In addition, strain $\varepsilon$ in the equation (1-1) is defined in 3.7 of the same standard, and is calculated by the following equation (1-2) specified in 10.2.1 "Strains determined with an extensometer" of the same standard.

$$E_t = (\sigma_2 - \sigma_1)/(\varepsilon_2 - \varepsilon_1) \qquad \dots \text{Equation (1-1)}$$

$$\varepsilon = \Delta L_0 / L_0 \qquad \dots \text{Equation (1-2)}$$

Note that, in equation (1-1), Et is elasticity (MPa), $\sigma_1$ is stress (MPa) at a time when strain $\varepsilon_1$ is 0.0005 (0.05%), and $\sigma_2$ is stress (MPa) at a time when strain $\varepsilon_2$ is 0.0025 (0.25%). In equation (1-2), $\varepsilon$ is the strain (%), $L_0$ is the gauge length (mm) of the test piece, and $\Delta L_0$ is an increment of the gauge length (mm) of the test piece.

It is more preferred that the adhesive supplementary marker 10 satisfy the following conditions 1-3 and 1-4.

> (Condition 1-3) The pressure-sensitive adhesive layer 12 has a thickness of 25 $\mu$m or less.
> (Condition 1-4) The adhesive supplementary marker body 10A has a peel strength of 1.5 N / 25 mm or more relative to a stainless steel test plate to which the pressure-sensitive adhesive layer 12 has been attached.

The peel strength is a value according to JIS Z 0237:2009. The peel strength is measured using a method according to "Testing methods of pressure-sensitive adhesive tapes and sheets" of JIS Z 0237:2009. The peel strength is measured as "Method 1: adhesive force against 180° peeling from test plate" in 10.4.1 of the same standard. When the adhesive supplementary marker body 10A satisfies the conditions 1-3 and 1-4, the adhesiveness of the pressure-sensitive adhesive layer 12 suppresses the formation of a gap between the breast and the pressure-sensitive adhesive layer 12. Thus, the smoothness of scans with a probe can be further increased.

The adhesive supplementary marker 10 further includes a protective film 13 laminated on the pressure-sensitive adhesive layer 12 on a side opposite to that on which the base film 11 is located, such that the protective film 13 is peelable from the pressure-sensitive adhesive layer 12. With this configuration, in the adhesive supplementary marker 10, the pressure-sensitive adhesive layer 12 is located between the base film 11 and the protective film 13. Thus, the pressure-sensitive adhesive layer 12 is kept clean until immediately before the adhesive supplementary marker 10 is used. In addition, between manufacture and use of the adhesive supplementary marker 10, the pressure-sensitive adhesive layer 12 is suppressed from unexpectedly adhering to other articles.

It is more preferred that the adhesive supplementary marker 10 satisfy the following condition 1-5.

(Condition 1-5) The adhesive supplementary marker body 10A has a transmittance of 70% or more to microwaves having a frequency of 2 GHz.

In mammography using microwaves, oscillating microwaves having a frequency of 2 GHz are emitted from the probe. When the adhesive supplementary marker body 10A satisfies condition (1-5), it is possible to more reliably generate three-dimensional images corresponding to a part of the breast to which the adhesive supplementary marker body 10A is attached. That is, it is possible to more reliably reproduce the part of the breast to which the adhesive supplementary marker body 10A is attached, in three-dimensional images. The adhesive supplementary marker body 10A is attached to the nipple portion during a mammography examination of the breast. When the adhesive supplementary marker body 10A satisfies condition 1-5, it is possible to more reliably generate three-dimensional images corresponding to the nipple portion and a part of a breast body continuous with the nipple portion.

Note that the transmittance of the adhesive supplementary marker body 10A to microwaves can be calculated from a ratio between reception intensity of the microwaves when the adhesive supplementary marker body 10A is not positioned between an oscillator and a receiver of the microwaves and reception intensity of the microwaves when the adhesive supplementary marker body 10A is positioned between the oscillator and the receiver of the microwaves.

It is more preferred that the adhesive supplementary marker 10 satisfy the following condition 1-6.

(Condition 1-6) The adhesive supplementary marker body 10A includes a part that has a total light transmittance of 30% or more and that enables visual recognition of the breast through the part of the adhesive supplementary marker body 10A.

The total light transmittance is a value according to JIS K 7361-1:1997. In other words, the total light transmittance is measured according to JIS K 7361-1:1997. In this embodiment, the entire adhesive supplementary marker body 10A has a total light transmittance of 30% or more. In other words, in this embodiment, at all parts of the adhesive supplementary marker body 10A, the total light transmittance is 30% or more. Note that only a part of the adhesive supplementary marker body 10A may have a total light transmittance of 30% or more and enable visual recognition of the breast through the part of the adhesive supplementary marker body 10A. In this case, the state of the part of the breast to which the adhesive supplementary marker body 10A is attached can be at least partially grasped through the adhesive supplementary marker body 10A. Note that, when the entire adhesive supplementary marker body 10A has a total light transmittance of 30% or more as in this embodiment, a state of the part of the breast to which the adhesive supplementary marker body 10A is attached can be grasped in its entirety through the adhesive supplementary marker body 10A.

The base film 11 may be made of various synthetic resins. Examples of such resins may include polyurethanes (PU), ethylene-vinyl acetate copolymers (EVA), polyethylene (PE), polypropylenes (PP), and polyethylene terephthalates (PET). Among these synthetic resins, the base film 11 is preferably made of PET. This allows the base film 11 to have a thickness of 20 $\mu$m or less while increasing the Young's modulus of the adhesive supplementary marker body 10A.

The base film 11 may be transparent or translucent. The entire base film 11 preferably has a total light transmittance of 30% or more.

The pressure-sensitive adhesive layer 12 may be made of various pressure-sensitive adhesives. The pressure-sensitive adhesive layer 12 may be made of a urethane-based pressure-sensitive adhesive. In this case, the pressure-sensitive adhesive layer 12 can have a thickness of 25 $\mu$m or less while allowing the adhesive supplementary marker body 10A to have a peel strength of 1.5 N / 25 mm or more. The pressure-sensitive adhesive layer 12 may be transparent or translucent. The entire pressure-sensitive adhesive layer 12 preferably has a total light transmittance of 30% or more.

The protective film 13 may be made of various synthetic resins. Examples of such resins may include PU, EVA, PE, PP, and PET. The protective film 13 preferably has higher stiffness than the base film 11. The protective film may be transparent or translucent. The entire protective film 13 preferably has a total light transmittance of 30% or more.

As illustrated in Fig. 2, as viewed from a viewpoint facing the plane in which the adhesive supplementary marker body 10A extends, the adhesive supplementary marker body 10A may have an elliptical shape or a capsule shape. In this embodiment, the adhesive supplementary marker body 10A has a capsule shape. Note that the capsule shape is also referred to as a racetrack shape. Additionally, as viewed from a viewpoint facing the plane in which the adhesive supplementary marker body 10A extends, the protective film 13 has the same shape as that of the adhesive supplementary marker body 10A extends. Thus, the adhesive supplementary marker 10 has the capsule shape. The protective film 13 may have a shape different from that of the adhesive supplementary marker body 10A. Note that, to keep the entire pressure-sensitive adhesive layer 12 clean, the protective film 13 is preferably shaped and sized to cover the entire pressure-sensitive adhesive layer 12.

The capsule shape refers to a shape formed by combining a quadrangle and two semicircles with each other, or a shape formed by combining a quadrangle and two semi-ellipses with each other. More specifically, the capsule shape refers to a shape formed by connecting two semicircles or two semi-ellipses to respective short sides of a quadrangle.

In this embodiment, the adhesive supplementary marker 10 has a shape formed by combining a rectangle and two semicircles with each other. Thus, a periphery 10E of the adhesive supplementary marker 10 is composed of two straight portions 10E1 and two arcuate portions 10E2. On the periphery 10E of the adhesive supplementary marker 10, the two straight portions 10E1 are arranged parallel to each other. Ends of the straight portions 10E1 on one side are connected to each other with one of the arcuate portions 10E2, and the ends of the straight portions 10E1 on the other side are connected to each other with the other of the arcuate portions 10E2.

A major axis AL and a minor axis AS of the adhesive supplementary marker body 10A each have a length of 45 mm or more. When the adhesive supplementary marker body 10A has the above capsule shape, the major axis AL is the longest segment among segments that are parallel to each of the straight portions 10E1 and touch the arcuate portions 10E2. In other words, the major axis AL is the longest segment among segments that are parallel to both the straight portions 10E1 and connect the two arcuate portions 10E2 to each other. The minor axis AS is a segment that is orthogonal to both the straight portions 10E1 and touches these two straight portions 10E1. In other words, the minor axis AS is a segment that is orthogonal to both the straight portions 10E1 and connects these two straight portions 10E1 to each other. Note that, when the adhesive supplementary marker body 10A has an elliptical shape, the major axis is the largest segment among segments that have both ends on a periphery of an ellipse. The minor axis is the largest segment among segments that have both ends on the periphery of the ellipse, and that are orthogonal to the major axis.

The ratio of the major axis AL to the minor axis AS (AL/AS) is referred to as an aspect ratio. In the adhesive supplementary marker body 10A, the aspect ratio is preferably greater than 1. In this case, when the examination target is the breast, and the adhesive supplementary marker body 10A is attached to the nipple portion, the adhesive supplementary marker body 10A is suppressed from becoming wrinkled. Thus, the smoothness of scans with a probe can be further increased.

[Adhesive Examination Marker Unit]

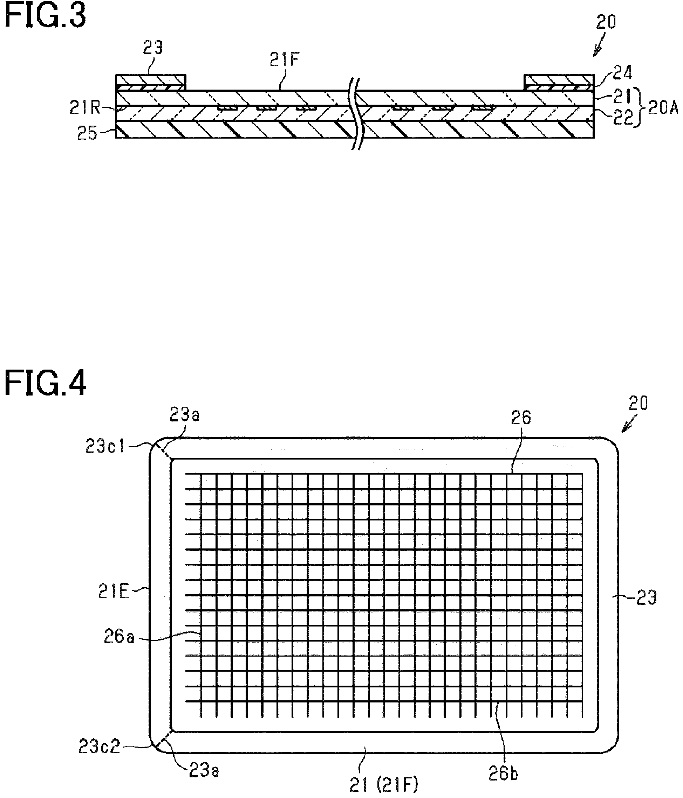

With reference to Figs. 3 and 4, the adhesive examination marker unit will be described. The adhesive examination marker unit includes the adhesive examination marker to be used in mammography using microwaves, and a support that supports the adhesive examination marker. With the adhesive supplementary marker attached to the breast, the adhesive examination marker is attached to the breast so that the adhesive examination marker covers the adhesive supplementary marker. The adhesive examination marker thus presses the adhesive supplementary marker, which is disposed between the adhesive examination marker and the breast, toward the breast.

As illustrated in Fig. 3, an adhesive examination marker unit 20 includes a base film 21, a pressure-sensitive adhesive layer 22, and a support 23. The base film 21 and the pressure-sensitive adhesive layer 22 constitute an adhesive examination marker 20A. The base film 21 has a front surface 21F and a rear surface 21R. The pressure-sensitive adhesive layer 22 is laminated on the rear surface 21R of the base film 21. The support 23 is attached to the front surface 21F of the base film 21.

The support 23 may be directly attached to the front surface 21F of the base film 21, or may be indirectly attached thereto. In this embodiment, the adhesive examination marker unit 20 further includes an adhesive layer 24 for allowing the support 23 to be attached to the front surface 21F of the base film 21. The support 23 is indirectly attached to the front surface 21F of the base film 21 via the adhesive layer 24.

The base film 21 is made of a synthetic resin. The base film 21 may be made, for example, of PU. As long as the adhesive examination marker 20A satisfies the following two conditions, the base film 21 may be made of synthetic resins other than PU. The base film 21 may have a thickness of, for example, 5 $\mu$m or more and 15 $\mu$m or less.

(Condition 2-1) The adhesive examination marker 20A has a tensile elongation at break of 130% or more.
(Condition 2-2) The adhesive examination marker 20A has tensile stress at 100% elongation of 10 MPa or less.

Since the adhesive examination marker 20A has a tensile elongation at break of 130% or more and a tensile stress at 100% elongation of 10 MPa or less, the adhesive examination marker 20A can be greatly elongated with a small force.

The tensile elongation at break is calculated according to "Plastics - Determination of tensile properties - Part 3: Test conditions for films and sheets" of JIS K 7127:1999 (ISO 527-3:1995), and according to "Plastics - Determination of tensile properties - Part 1: General principles" of JIS K 7161-1:2014 (ISO 527-1:2012).

When a test piece that is an examination target does not have a yield point, the "strain at break" defined in 3.7.2 of JIS K 7161-1:2014 is calculated as the tensile elongation at break. On the other hand, when the test piece has a yield point, the "nominal strain at break" defined in 3.8.1 of the same standard is calculated as the tensile elongation at break. Note that the strain at break is calculated by the following equation (2-1) specified in 10 "Calculation and expression of results" of the same standard. Further, the nominal strain at break is calculated by the following equation (2-2) specified in 10 "Calculation and expression of results" of the same standard.

$$\varepsilon = \Delta L_0 / L_0 \qquad \dots \text{Equation (2-1)}$$

$$\varepsilon_t = \varepsilon_y + \Delta L_t / L \qquad \dots \text{Equation (2-2)}$$

In the equation (2-1), $\varepsilon$ is the strain (%), $L_0$ is the gauge length (mm) of the test piece, and $\Delta L_0$ is an increment of the gauge length (mm) of the test piece. In the equation (2-2), $\varepsilon_t$ is nominal strain (%), $\varepsilon_y$ is yield strain (%), L is an initial distance (mm) between gripping jaws, and $\Delta L_t$ is an increment of the distance (mm) between the gripping jaws from the yield point.

The tensile stress at 100% elongation is calculated using a method according to "Plastics - Determination of tensile properties - Part 3: Test conditions for films and sheets" of JIS K 7127:1999 (ISO 527-3:1995). In addition, the tensile stress at 100% elongation is calculated as stress at a time when strain reaches a predetermined value (100%), according to "stress at x% strain" defined in 3.6.3 in "Plastics - Determination of tensile properties - Part 1: General principles" of JIS K 7161-1:2014 (ISO 527-1:2012). Note that the stress at 100% strain is calculated by the following equation (2-3) specified in 10 "Calculation and expression of results" of the same standard.

$$\sigma = F / A \dots \text{Equation (2-3)}$$

Note that, in the equation (2-3), $\sigma$ is stress (MPa), F is measured force (N), and A is an initial cross-sectional area (mm$^2$) of the test piece.

When the base film 21 is made of PU, it is preferred that PU contain at least one selected from a group of ether-based PU, ester-based PU, and carbonate-based PU. In this case, the base film 21 obtained can be highly flexible and easily attached to the examination target. The ether-based PU refers to PU produced using an ether-based polyol containing an ether bond (-O-). The ester-based PU is PU produced using an ester-based polyol containing an ester bond (-COO-). The carbonate-based PU is PU produced using a polyol containing a carbonate bond (-OC(=O)O-).

Similarly to the base film 21, the pressure-sensitive adhesive layer 22 is made of a synthetic resin. The pressure-sensitive adhesive layer 22 may be made of PU. As long as the adhesive examination marker 20A satisfies not only the above conditions 2-1 and 2-2, but also the following condition 2-3, the pressure-sensitive adhesive layer 22 may be made of synthetic resins other than PU. The pressure-sensitive adhesive layer 22 may have a thickness of, for example, 5 μm or more and 25 μm or less.

(Condition 2-3) The adhesive examination marker 20A has a water vapor transmission rate according to JIS Z 0208-1976 of 750 g/m²/day or more under conditions of 40°C and 90% RH.

The adhesive examination marker 20A more preferably satisfies the following condition 2-4.

(Condition 2-4) The adhesive examination marker 20A has a transmittance of 70% or more to microwaves having a frequency of 2 GHz.

This configuration increases reliability of generating three-dimensional images corresponding to a part to which the adhesive examination marker 20A is attached. Note that the transmittance of the adhesive examination marker 20A to microwaves can be calculated from a ratio between reception intensity of the microwaves when the adhesive examination marker 20A is not disposed between an oscillator and a receiver of the microwaves and reception intensity of the microwaves when the adhesive examination marker 20A is disposed between the oscillator and the receiver of the microwaves.

The support 23 may be made of paper or a synthetic resin. The support 23 preferably has higher stiffness than the adhesive examination marker 20A. The support 23 also preferably has flexibility that enables the adhesive examination marker unit 20 to curve along a curved surface of, for example, the breast, when the adhesive examination marker unit 20 is applied to such a curved surface. When the support 23 is made of paper, the stiffness and the flexibility of the support 23 can be adjusted by adjusting the basis weight of the paper, that is, the weight per unit area of the same. When the support 23 is made of a synthetic resin, the stiffness and the flexibility of the support 23 can be adjusted by changing the type of a synthetic resin and by adjusting the thickness of the support 23.

The adhesive examination marker unit 20 further includes a protective film 25. The protective film 25 is laminated on the pressure-sensitive adhesive layer 22 in such a manner as to be peelable therefrom. As viewed from a viewpoint facing the protective film 25, the protective film 25 covers the entire pressure-sensitive adhesive layer 22. The protective film 25 is preferably made of a transparent or translucent synthetic resin. The protective film 25 may include a substrate film and a release layer. The release layer is laminated on the substrate film. The release layer of the protective film 25 is in contact with the pressure-sensitive adhesive layer 22. The substrate film may be, for example, a PET film. The release layer may be, for example, a silicone resin layer. Note that the protective film 25 may be comprised only of the substrate film, and a surface of the substrate film in contact with the pressure-sensitive adhesive layer 22 may be processed to have higher peelability.

As illustrated in Fig. 4, as viewed from a viewpoint facing the front surface 21F of the base film 21, the support 23 has a shape extending along the edge 21E of the base film 21. The adhesive examination marker 20A is supported by the support 23, whereby a portion of the edge of the adhesive examination marker 20A supported by the support 23 is maintained in a stretched state while conforming to the shape of the support 23. Therefore, the technician who attaches the adhesive examination marker 20A to the breast can maintain the entire adhesive examination marker 20A without wrinkles by only pulling outward portions of the edge of the adhesive examination marker 20A that are not supported by the support 23. Accordingly, the adhesive examination marker 20A can be attached to the breast without wrinkles; hence, the adhesive examination marker 20A can be suppressed from becoming wrinkled when attached to the breast.

The base film 21 includes a coordinate grid 26 for guiding a scanning position on the breast. As viewed from a viewpoint facing the front surface 21F of the base film 21, the coordinate grid 26 is located in a region surrounded by the support 23. The coordinate grid 26 includes a plurality of first grid lines 26a. Each first grid line 26a extends along a scanning direction, and the plurality of first grid lines 26a are arranged in an array direction crossing the scanning direction. In the present embodiment, the vertical direction of the drawing sheet corresponds to the scanning direction, and the horizontal direction of the drawing sheet corresponds to the array direction. The scanning direction in mammography refers to a direction in which the technician scans the examination target with a probe.

The coordinate grid 26 further includes a plurality of second grid lines 26b extending along the array direction and arranged in the scanning direction. As viewed from a viewpoint facing the front surface 21F of the base film 21, the plurality of second grid lines 26b form a square grid together with the plurality of first grid lines 26a.

The coordinate grid 26 is printed on the rear surface 21R of the base film 21 using ink. As the ink for printing the coordinate grid 26, any ink that can be printed on the base film 21 can be used.

In the adhesive examination marker unit 20, the above total light transmittance is preferably 30% or more in parts of the adhesive examination marker 20A except the coordinate grid 26. In this case, when the adhesive examination marker unit 20 is attached to the breast, it is possible to adjust the position of the adhesive examination marker unit 20 relative to the breast while visually checking the position of the coordinate grid 26 relative to the breast. Moreover, when the total light transmittance of the adhesive examination marker unit 20 is 30% or more, after the base film 21 and the pressure-sensitive adhesive layer 22 are attached to the breast, the position of moles and spots on the breast can be identified through them visually or using a camera. For example, the positions of moles and spots on the breast can be identified by the technician, or can be identified using detection equipment including a camera. Since the position of moles and spots on the breast does not change, the positions of moles and spots on the breast are important in identifying the position of any lesion in the breast.

**In** contrast, the above total light transmittance of the support 23 is preferably lower than that of the parts of the adhesive examination marker 20A except the coordinate grid 26. The support 23 is preferably translucent or opaque. This makes the boundary between the adhesive examination marker 20A and the support 23 clearer than when the support 23 has the same or substantially the same degree of transparency as the adhesive examination marker 20A. Thus, when removing the support 23 from the adhesive examination marker unit 20, it is easy to determine whether the adhesive examination marker 20A is torn along the support 23. This prevents inadvertent tearing inside the boundary between the adhesive examination marker 20A and the support 23 when removing the support 23 from the adhesive examination marker unit 20.

The support 23 includes a weakened portion 23a. The weakened portion 23a is a portion of the support 23 that is more easily broken than its portions other than the weakened portion 23a. The weakened portion 23a has lower mechanical strength than portions of the support 23 other than the weakened portion 23a. The weakened portion 23a allows the support 23 to be broken starting from the weakened portion 23a. Thus, it is easy to remove the support 23 from the front surface 21F of the base film 21.

The breaking strength of the portions of the support 23 other than the weakened portion 23a is higher than that of the base film 21, and the adhesion strength between the support 23 and the front surface 21F of the base film 21 is higher than that between the pressure-sensitive adhesive layer 22 and the examination target. Thus, when the support 23 is broken starting from the weakened portion 23a, the adhesive examination marker 20A can be broken along the boundary between the support 23 and the base film 21 as viewed from a viewpoint facing the front surface 21F of the base film 21. Further, portions of the adhesive examination marker 20A that overlap the support 23 as viewed in the thickness direction of the adhesive examination marker 20A can be removed from the breast together with the support 23.

As viewed from a viewpoint facing the front surface 21F of the base film 21, the weakened portion 23a is preferably disposed in an area of the support 23 including a portion with the largest distance from the center of gravity of the base film 21. **In** this case, compared with the case where the distance between the weakened portion 23a and the center of gravity of the base film 21 is smaller, wrinkling of the base film 21 and change in position of the base film 21 relative to the breast can be suppressed when the support 23 is removed from the base film 21.

**In** the present embodiment, as viewed from a viewpoint facing the front surface 21F of the base film 21, the adhesive examination marker 20A has a rectangular shape, and the support 23 has a rectangular frame shape. Therefore, the portions of the support 23 with the largest distance from the center of gravity of the base film 21 are corner portions of the support 23. The support 23 includes a first corner portion 23c1, and a second corner portion 23c2 adjacent to the first corner portion 23c1. The support 23 preferably includes the weakened portion 23a in at least one of the first corner portion 23c1 and the second corner portion 23c2. In this case, compared with the case where the weakened portion 23a is disposed in a portion of the support 23 except the corner portions, it is easier to hold portions of the support 23 around the weakened portion 23a, and it is thus easier to apply a force to the weakened portion 23a to break the weakened portion 23a.

Moreover, in the present embodiment, the weakened portion 23a is disposed in both the first corner portion 23c1 and the second corner portion 23c2. This increases the degree of freedom in attaching the adhesive examination marker unit 20 to the examination target, compared with the case where the support 23 has only one weakened portion 23a. In particular, when the examination target is two symmetric sites, such as breasts, providing the weakened portion 23a to both the first corner portion 23c1 and the second corner portion 23c2 makes it possible to obtain effects of the weakened portion 23a in both the left and right breasts.

[Usage]

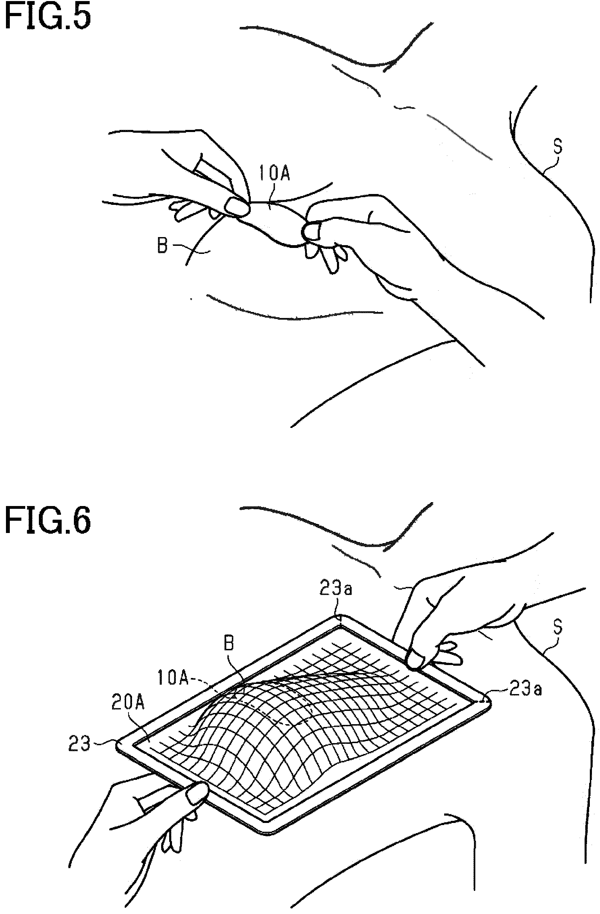

With reference to Figs. 5 to 7, the usage of the adhesive supplementary marker and the adhesive examination marker unit will be described.

As illustrated in Fig. 5, when using the adhesive supplementary marker 10 and the adhesive examination marker unit 20, first, the adhesive supplementary marker body 10A is attached to a subject S. This attachment operation is performed with the protective film 13 of the adhesive supplementary marker 10 released from the pressure-sensitive adhesive layer 12. Then, the adhesive supplementary marker body 10A is attached to a breast B of the subject S so that the adhesive supplementary marker body 10A covers a nipple portion of the breast B. Note that, in the example illustrated in Fig. 5, the adhesive supplementary marker body 10A is attached to the breast B so that a direction in which the major axis of the adhesive supplementary marker body 10A extends is substantially parallel to a lateral direction of the subject S. However, the adhesive supplementary marker body 10A may be attached to the breast B so that the direction in which the major axis of the adhesive supplementary marker body 10A extends intersects with the lateral direction of the subject S.

As illustrated in Fig. 6, with the adhesive supplementary marker body 10A attached to the breast B, the adhesive examination marker 20A is attached to the breast B so that the adhesive examination marker 20A covers the adhesive supplementary marker body 10A. This operation is performed with the protective film 25 of the adhesive examination marker unit 20 released from the pressure-sensitive adhesive layer 22. Then, while the support 23 is being held in such a manner that the adhesive examination marker 20A does not become wrinkled, the pressure-sensitive adhesive layer 22 of the adhesive examination marker 20A is attached to the breast B. In this way, the pressure-sensitive adhesive layer 22 of the adhesive examination marker 20A is attached to the adhesive supplementary marker body 10A and the breast body. Thus, adhesive force of the pressure-sensitive adhesive layer 22 causes the adhesive supplementary marker body 10A to be pressed against the breast B.

The adhesive examination marker 20A is attached to the breast B with a level difference between the nipple portion and the breast body reduced by the adhesive supplementary marker body 10A. Thus, when a scan is performed with a probe along the adhesive examination marker 20A, scanning with the probe is suppressed from being obstructed by the nipple portion. Accordingly, scans with a probe can be performed more smoothly.

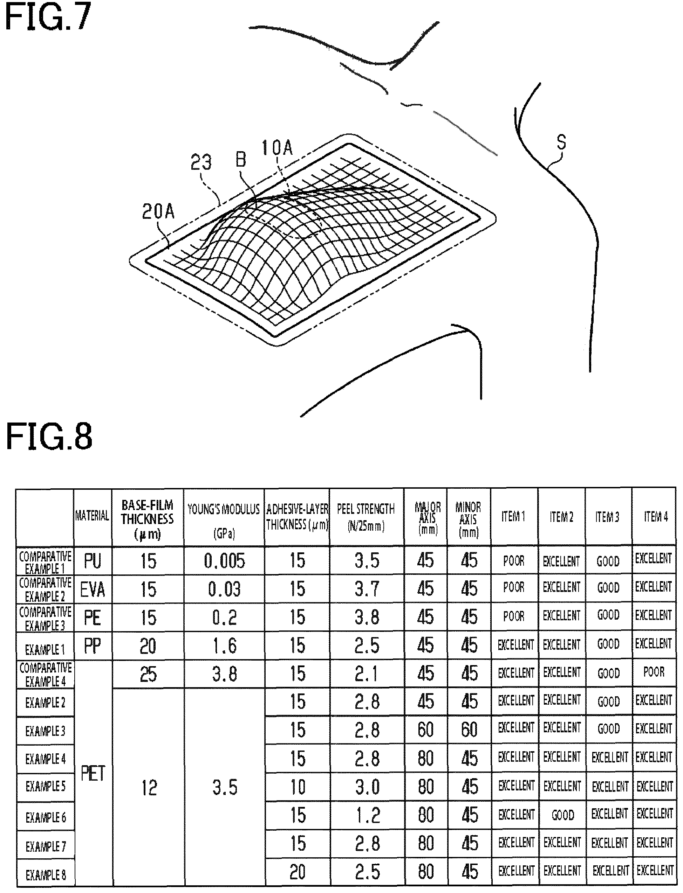

As illustrated in Fig. 7, parts of the adhesive examination marker 20A attached to the breast B and the support 23 are removed from the breast B. In this removal operation, first, the technician breaks the support 23 along the weakened portions 23a simultaneously with breaking the parts of the adhesive examination marker 20A above which the weakened portions 23a are located. Then, as viewed from a viewpoint facing a plane in which the adhesive examination marker 20A extends, the examiner breaks the adhesive examination marker 20A along the boundary between the adhesive examination marker 20A and the support 23. In this way, the support 23 can be removed from the breast B together with the parts of the adhesive examination marker 20A.

[Examples]

With reference to Fig. 8, Examples and Comparative Examples of the adhesive supplementary marker will be described.

[Comparative Example 1]

A 15-μm thick PU film made of ether-based PU (SILKLON HVL85 manufactured by Okura Industrial Co., Ltd.) (SILKLON is a registered trademark) was prepared as a base film. Note that a separation film had previously been laminated on the base film. A curing agent (T-501B manufactured by Toyochem Co., Ltd.) was added to a urethane-based pressure-sensitive adhesive (SP-205 manufactured by Toyochem Co., Ltd.) as a base resin, and the resulting mixture was stirred to prepare a coating liquid. The curing agent was added to the base resin so that a ratio of the number of moles of isocyanate groups contained in the curing agent to the number of moles of hydroxyl groups contained in the base resin would be 0.13. The coating liquid was then applied to a protective film (Cerapeel WZ manufactured by Toray Advanced Film Co., Ltd.) by gravure coating, followed by drying. Thus, a pressure-sensitive adhesive layer having a thickness of 15 μm was obtained. The base film and the pressure-sensitive adhesive layer obtained were laminated on each other to obtain a laminate. The separation film was released from the base film, and then the laminate including the base film, pressure-sensitive adhesive layer, and protective film was cut into a shape of a circle having a diameter of 45 mm. an adhesive supplementary marker of Comparative Example 1 was thus obtained.

[Comparative Example 2]

An adhesive supplementary marker of Comparative Example 2 was obtained using the same method as that in Comparative Example 1 except that an EVA film having a thickness of 15 μm was formed as a base film by subjecting EVA (Novatec LV400 manufactured by Japan Polyethylene Corporation) (Novatec is a registered trademark) to extrusion lamination, using a separation film (Cerapeel BX-9 manufactured by Toray Advanced Film Co., Ltd.) as a support.

[Comparative Example 3]

An adhesive supplementary marker of Comparative Example 3 was obtained using the same method as that in Comparative Example 2 except that a PE film having a thickness of 15 μm was formed as a base film by subjecting PE pellets (LC600A manufactured by Japan Polyethylene Corporation) to extrusion lamination, using the abovementioned separation film as a support.

[Example 1]

An adhesive supplementary marker of Example 1 was obtained using the same method as that in Comparative Example 1 except that an OPP (Oriented Polypropylene) film (FOR-M manufactured by Futamura Chemical Co.,Ltd.) having a thickness of 20 μm was prepared as a base film.

[Comparative Example 4]

An adhesive supplementary marker of Comparative Example 4 was obtained using the same method as that in Comparative Example 1 except that a PET film (FE2000 manufactured by Futamura Chemical Co.,Ltd.) having a thickness of 25 μm was prepared as a base film.

[Example 2]

An adhesive supplementary marker of Example 2 was obtained using the same method as that in Comparative Example 1 except that a PET film (FE3001 manufactured by Futamura Chemical Co.,Ltd.) having a thickness of 12 μm was prepared as a base film.

[Example 3]

An adhesive supplementary marker of Example 3 was obtained using the same method as that in Example 2 except that the laminate including the base film, pressure-sensitive adhesive layer, and protective film was cut into a shape of a circle having a diameter of 60 mm.

[Example 4]

An adhesive supplementary marker of Example 4 was obtained using the same method as that in Example 2 except that the laminate including the base film, pressure-sensitive adhesive layer, and protective film was cut into a capsule shape having a major axis of 80 mm and a minor axis of 45 mm.

[Example 5]

An adhesive supplementary marker of Example 5 was obtained using the same method as that in Example 2 except that the thickness of the pressure-sensitive adhesive layer was changed to 10 μm, and that the laminate including the base film, pressure-sensitive adhesive layer, and protective film was cut into a capsule shape having a major axis of 80 mm and a minor axis of 45 mm in Example 2.

[Example 6]

An adhesive supplementary marker of Example 6 was obtained using the same method as that in Example 5 except that a ratio of the number of moles of isocyanate groups contained in the curing agent to the number of moles of hydroxyl groups contained in the base resin was changed to 0.25, and that the thickness of the pressure-sensitive adhesive layer was changed to 15 μm.

[Example 7]

An adhesive supplementary marker of Example 7 was obtained using the same method as that in Example 6 except that a ratio of the number of moles of isocyanate groups contained in the curing agent to the number of moles of hydroxyl groups contained in the base resin was changed to 0.13.

[Example 8]

An adhesive supplementary marker of Example 8 was obtained using the same method as that in Example 7 except that the thickness of the pressure-sensitive adhesive layer was changed to 20 μm.

[Evaluation Method]

[Young's Modulus]

The base films of the respective adhesive supplementary markers were punched into a dumbbell shape (test piece of Type 5) according to "Plastics - Determination of tensile properties - Part 3: Test conditions for films and sheets" of JIS K 7127:1999. Then, the Young's modulus of the base films was measured using a method according to "Plastics - Determination of tensile properties - Part 1: General principles" of JIS K 7161-1:2014. For this measurement, a tensile testing machine (Autograph AGS-X with a 1-kN load cell, the Autograph AGS-X being manufactured by Shimadzu Corporation.) was used with its test speed set to 300 mm/min. Note that the base films of the respective adhesive supplementary markers were prepared as targets for the measurement of the Young's modulus.

[Peel Strength]

The peel strength of each of the adhesive supplementary marker bodies was measured using the method according to "Testing methods of pressure-sensitive adhesive tapes and sheets" of JIS Z 0237:2009. For this measurement, the respective adhesive supplementary markers were used to form their test pieces each having a width of 25 mm. Then, the protective films of test pieces were released from their pressure-sensitive adhesive layers, and these adhesive layers were attached to respective stainless steel plates. Next, a load was applied to each of the test pieces by causing a 2-kg roller to reciprocate twice over the test pieces, and the test pieces were left for 30 minutes or more. Subsequently, the tensile testing machine (Autograph AGS-X with a 1-kN load cell, the Autograph AGS-X being manufactured by Shimadzu Corporation.) was used to measure the peel strength of each adhesive supplementary marker body when the test pieces were peeled away from the respective stainless steel test plates at an angle of 180° and at a peeling speed of 300 mm/min.

[Attachment Suitability]

Attachment suitability was evaluated on the following four items using a breast-cancer palpation training model (manufactured by TANAC Co., Ltd.).

(Item 1) The adhesive supplementary marker body is capable of pressing the nipple portion in a state in which the adhesive supplementary marker body is attached to the breast.

(Item 2) The adhesive supplementary marker body is suppressed from being lifted from the breast in a state in which the adhesive supplementary marker body is attached to the breast.

(Item 3) The adhesive supplementary marker body is suppressed from becoming wrinkled in a state in which the adhesive supplementary marker body is attached to the breast.

(Item 4) A level difference at the surface of the adhesive examination marker is suppressed in a state in which the adhesive supplementary marker body is attached to the breast with the adhesive examination marker attached from above the adhesive supplementary marker body.

As Item 1, whether a level difference between the nipple portion and the breast body was reduced in a state in which the adhesive supplementary marker body of each of the adhesive supplementary markers was attached to the nipple portion was evaluated on a scale of the following three levels:

Excellent: There is no level difference between the nipple portion and the breast body, or there is so small a level difference therebetween that scans with a probe are not obstructed.
Good: There is a level difference between the nipple portion and the breast body in a state in which only the adhesive supplementary marker body is attached, but there is no level difference therebetween or there is so small a level difference therebetween that scans with a probe are not obstructed, in a state in which the adhesive examination marker is attached from above the adhesive supplementary marker body.
Poor: There is so large a level difference between the nipple portion and the breast body that scans with a probe are obstructed, in a state in which only the adhesive supplementary marker body is attached and in a state in which the adhesive examination marker is attached from above the adhesive supplementary marker body.

As Item 2, whether the adhesive supplementary marker body of each of the adhesive supplementary markers was suppressed from being lifted from the breast in a state in which the adhesive supplementary marker body was attached to the nipple portion was evaluated on a scale of the following three levels.

Excellent: The edge of the adhesive supplementary marker body adheres to the breast, and there is no space between the adhesive supplementary marker body and the breast within the edge of the adhesive supplementary marker body or there is so small a space therebetween that scans with a probe are not obstructed.

Good: There is a space between the adhesive supplementary marker body and the breast within the edge of the adhesive supplementary marker body when only the adhesive supplementary marker body is attached to the breast, but there is no space between the adhesive supplementary marker body and the breast within the edge or there is so small a space therebetween that scans with a probe are not obstructed, in a state in which the adhesive examination marker is attached from above the adhesive supplementary marker body.

Poor: There is so large a space between the adhesive supplementary marker body and the breast within the edge of the adhesive supplementary marker body that scans with a probe are obstructed, when only the adhesive supplementary marker body is attached and when the adhesive examination marker is attached from above the adhesive supplementary marker body.

As Item 3, whether the adhesive supplementary marker was suppressed from becoming wrinkled in a state in which the adhesive supplementary marker body of each of the adhesive supplementary markers was attached to the nipple portion was evaluated on a scale of the following three levels.

Excellent: The adhesive supplementary marker body does not become wrinkled, or becomes wrinkled only to such a small extent that scans with a probe are not obstructed.

Good: The adhesive supplementary marker body becomes wrinkled in a state in which only the adhesive supplementary marker body is attached, but in a state in which the adhesive examination marker is attached from above the adhesive supplementary marker body, wrinkling of the adhesive supplementary marker body does not cause the adhesive examination marker to become wrinkled, or causes the adhesive examination marker to become wrinkled only to such a small extent that scans with a probe are not obstructed.

Poor: Wrinkling of the adhesive supplementary marker body causes the adhesive examination marker to become wrinkled to such a large extent that scans with a probe are obstructed, when the adhesive examination marker is attached from above the adhesive supplementary marker body.

As Item 4, whether a level difference at the surface of the adhesive examination marker was suppressed in a state in which the adhesive supplementary marker body of each of the adhesive supplementary markers was attached to the nipple portion and the adhesive examination marker was attached from above the adhesive supplementary marker body was evaluated on a scale of the following two levels.

Excellent: There is no level difference at the surface of the adhesive examination marker, or there is such a small extent of level difference thereat that scans with a probe are obstructed.

Poor: There is such a large extent of level difference at the surface of the adhesive examination marker that scans with a probe are not obstructed.

[Evaluation Results]

With reference to Fig. 8, the evaluation results will be described.

As shown in Fig. 8, with regard to Item 1, the adhesive supplementary markers of Comparative Examples 1 to 3 were evaluated as "Poor", whereas the adhesive supplementary markers of Comparative Example 4 and Examples 1 to 8 were evaluated as "Excellent". With regard to Item 4, the adhesive supplementary markers of Comparative Examples 1 to 3 and Examples 1 to 8 were evaluated as "Excellent", whereas Comparative Example 4 was evaluated as "Poor".

Thus, when an adhesive supplementary marker body has a Young's modulus of 1.0 GPa or more, and the base film thereof has a thickness of 20 μm or less, the adhesive supplementary marker body can adhere to the breast in such a manner as to reduce a level difference between the breast and the nipple portion. As a result, scans using a probe can be performed more smoothly.

Further, with regard to Item 2, the adhesive supplementary markers of Comparative Examples 1 to 4 and Examples 1 to 5 and Example 7 were evaluated as "Excellent", whereas the adhesive supplementary marker of Example 6 was evaluated as "Good". Thus, when an adhesive supplementary marker body has a peel strength of 1.5 N / 25 mm or more, the adhesive supplementary marker body can more easily adhere to the breast. As a result, the smoothness of scans with a probe can be further increased.

Still further, with regard to Item 3, the adhesive supplementary markers of Comparative Examples 1 to 4 and Examples 1 to 3 were evaluated as "Good", whereas the adhesive supplementary markers of Examples 4 to 8 were evaluated as "Excellent". Thus, when an adhesive supplementary marker body has the above elliptical or capsule shape and has a minor axis of 45 mm or more, an adhesive supplementary marker is suppressed from becoming wrinkled. As a result, the smoothness of scans with a probe can be further increased.

As described above, the present embodiment of the adhesive supplementary marker can achieve the following advantageous effects.

(1) In a state in which an adhesive supplementary marker body 10A is attached to the breast B so that the adhesive supplementary marker body 10A covers the nipple portion of the breast B, the Young's modulus of the adhesive supplementary marker body 10A enables the adhesive supplementary marker body 10A to adhere to the breast B while pressing the nipple portion in the direction in which the height of the nipple portion is reduced. This enables the adhesive supplementary marker 10 to reduce the level difference of the breast B. Thus, scans with a probe can be performed more smoothly.

(2) The adhesiveness of the pressure-sensitive adhesive layer 12 suppresses the formation of a gap between the breast B and the pressure-sensitive adhesive layer 12. Thus, the smoothness of scans with a probe can be further increased.

(3) When the examination target is the breast, and the adhesive supplementary marker body 10A is attached to the nipple portion, the adhesive supplementary marker body 10A is suppressed from becoming wrinkled. Thus, the smoothness of scans with a probe can be further increased.

(4) The pressure-sensitive adhesive layer 12 is located between the base film 11 and the protective film 13. Thus, the pressure-sensitive adhesive layer 12 is kept clean until immediately before the adhesive supplementary marker 10 is used.

(5) It is possible to more reliably generate three-dimensional images corresponding to the part of the breast B to which the adhesive supplementary marker body 10A is attached.

(6) The state of the part of the breast B to which the adhesive supplementary marker body 10A is attached can be at least partially grasped through the adhesive supplementary marker body 10A.

The embodiment described above may be modified and implemented as follows.

[Total Light Transmittance]

- The entire adhesive supplementary marker body 10A may have a total light transmittance of less than 30%. Also in this case, the same or substantially the same effect as in (1) above can be obtained, as long as the base film 11 has a thickness of 20 μm or less, and the adhesive supplementary marker body 10A has a Young's modulus of 1.0 GPa or more.

[Microwave Transmittance]

- The transmittance of the adhesive supplementary marker body 10A to microwaves having a frequency of 2 GHz may be less than 70%. Also in this case, the same or substantially the same effect as in (1) above can be obtained, as long as the base film 11 has a thickness of 20 μm or less, and the adhesive supplementary marker body 10A has a Young's modulus of 1.0 GPa or more.

[Protective Film]

- The adhesive supplementary marker 10 may not include the protective film 13. Also in this case, the same or substantially the same effect as in (1) above can be obtained, as long as the base film 11 has a thickness of 20 μm or less, and the adhesive supplementary marker body 10A has a Young's modulus of 1.0 GPa or more.

[Shape of Adhesive Supplementary Marker Body]

As viewed from a viewpoint facing the plane in which the adhesive supplementary marker body 10A extends, the adhesive supplementary marker body 10A may have a shape other than an elliptical shape and a capsule shape. The adhesive supplementary marker body 10A may have, for example, a circular shape as mentioned in Examples. Alternatively, the adhesive supplementary marker body 10A may have a polygonal shape such as a rectangular shape. Note that the adhesive supplementary marker body 10A preferably has any of an elliptical shape, a capsule shape, and a circular shape because, during scanning with a probe along the adhesive examination marker 20A attached to the breast from above the adhesive supplementary marker body 10A, any other shape with corner portions may cause collision of the probe with the adhesive examination marker 20A at positions corresponding to the corner portions of the adhesive supplementary marker body 10A, and the above shapes suppress such a collision.

[Pressure-sensitive Adhesive Layer]

The pressure-sensitive adhesive layer 12 may have a thickness of more than 25 μm. Also in this case, the same or substantially the same effect as in (1) above can be obtained, as long as the base film 11 has a thickness of 20 μm or less, and the adhesive supplementary marker body 10A has a Young's modulus of 1.0 GPa or more.

[Peel Strength]

The adhesive supplementary marker body 10A may have a peel strength of less than 1.5 N / 25 mm. Also in this case, the same or substantially the same effect as in (1) above can be obtained, as long as the base film 11 has a thickness of 20 μm or less, and the adhesive supplementary marker body 10A has a Young's modulus of 1.0 GPa or more.

[Adhesive Examination Marker Unit]

- The support 23 may not have a shape extending along the entire edge 21E of the base film 21 and may instead have a shape extending along only a part of the edge 21E. The support 23 may have, for example, a U-shape or an L-shape as viewed from a viewpoint facing the front surface 21F of the base film 21.

As viewed from a viewpoint facing the front surface 21F of the base film 21, the adhesive examination marker 20A may have a shape other than a rectangular shape. The adhesive examination marker 20A may have, for example, a polygonal shape other than a rectangular shape, a circular shape, or an elliptical shape.

[Application Target]

- An application target of the adhesive supplementary marker 10, that is, the examination target of image diagnosis using microwaves, is not limited to the breast described above and may be another part of a human body having a level difference due to a protruding portion located on a surface of the examination target. Also in this case, the adhesive supplementary marker 10 is capable of providing the same or substantially the same effects as those in the case where the examination target is the breast.

**Claims**

1.  An adhesive examination marker set comprising:

    an adhesive supplementary marker (10) configured to be used together with an adhesive examination marker to be attached to an examination target of image diagnosis using microwaves, and that is configured to be disposed between the adhesive examination marker and the examination target and to be pressed toward the examination target by the adhesive examination marker in a state in which the adhesive examination marker is attached to the examination target, the adhesive supplementary marker comprising a laminate including a base film having a thickness of 20 μm or less, and a pressure-sensitive adhesive layer configured to be attached to the examination target, the base film having a Young's modulus of 1.0 GPa or more; **characterised in that** the adhesive examination marker set further comprises an adhesive examination marker (20A) configured to be attached to the examination target so that the adhesive examination marker (20A) covers the adhesive supplementary marker (10) attached to the examination target, wherein the adhesive supplementary marker (10) is configured to be used together with the adhesive examination marker and is configured to be disposed between the adhesive examination marker (20A) and the examination target and to be pressed toward the examination target by the adhesive examination marker (20A) in a state in which the adhesive examination marker

(20A) is attached to the examination target.

2. The adhesive examination marker set according to claim 1, wherein

> the pressure-sensitive adhesive layer (12) has a thickness of 25 μm or less,
> the laminate has a peel strength of 1.5 N / 25 mm or more relative to a stainless steel test plate to which the pressure-sensitive adhesive layer (12) has been attached, the peel strength being measured according to JIS Z 0237:2009.

3. The adhesive examination marker set according to claim 1 or 2, wherein, as viewed from a viewpoint facing a plane in which the laminate extends,

> the laminate has an elliptical shape or a capsule shape,
> a major axis and a minor axis of the laminate each have a length of 45 mm or more, and
> a ratio of the major axis to the minor axis is greater than 1.

4. The adhesive examination marker set according to any one of claims 1 to 3, further comprising a protective film (13) laminated on a surface of the pressure-sensitive adhesive layer (12) on a side opposite to that facing the base film (11), the protective film (13) being peelable from the pressure-sensitive adhesive layer (12).

5. The adhesive examination marker set according to any one of claims 1 to 4, wherein the laminate has a transmittance of 70% or more to microwaves having a frequency of 2 GHz.

6. The adhesive examination marker set according to any one of claims 1 to 5, wherein the laminate includes a part that enables visual recognition of the examination target through the part of laminate, the part of the laminate having a total light transmittance of 30% or more, the total light transmittance being measured according to JIS K 7361-1:1997.

**Patentansprüche**

1. Satz von klebenden Untersuchungsmarkern, umfassend:

> einem klebenden Zusatzmarker (10), der dazu ausgelegt ist, zusammen mit einem selbstklebenden Untersuchungsmarker verwendet zu werden, der an einem Untersuchungsobjekt der Bilddiagnose mittels Mikrowellen angebracht wird, und der dafür ausgelegt ist, dass er zwischen dem selbstklebenden Untersuchungsmarker und dem Untersuchungsobjekt angeordnet wird und in einem Zustand in dem der klebende Untersuchungsmarker am Untersuchungsobjekt angebracht ist durch den selbstklebenden Untersuchungsmarker in Richtung des Untersuchungsobjekts gedrückt wird, wobei die klebende Zusatzmarkierung ein Laminat umfasst, das einen Trägerüberzug mit einer Dicke von 20 μm oder weniger und eine druckempfindliche Klebeschicht enthält, die dazu ausgelegt ist, am Untersuchungsobjekt befestigt zu werden, wobei der Trägerüberzug einen Elastizitätsmodul von 1,0 GPa oder mehr aufweist;

**dadurch gekennzeichnet, dass** der Satz von klebenden Untersuchungsmarkern ferner umfasst:

> einen klebenden Untersuchungsmarker (20A) umfasst, der so ausgebildet ist, dass er an dem Prüfobjekt befestigt wird, sodass der klebende Untersuchungsmarker (20A) den an dem Prüfobjekt befestigten klebenden Zusatzmarker (10) bedeckt,
> wobei der klebende Zusatzmarker (10) so ausgebildet ist, dass er zusammen mit dem klebenden Untersuchungsmarker verwendet wird, und, wenn der klebende Untersuchungsmarker (20A) am Prüfobjekt angebracht ist, so ausgebildet ist, dass er zwischen dem klebenden Untersuchungsmarker (20A) und dem Prüfobjekt angeordnet und durch den klebenden Untersuchungsmarker (20A) in Richtung des Prüfobjekts gedrückt wird.

2. Satz von klebenden Untersuchungsmarkern nach Anspruch 1, wobei

> die druckempfindliche Klebeschicht (12) eine Dicke von 25 μm oder weniger aufweist,
> das Laminat eine Schälfestigkeit von 1,5 N/25 mm oder mehr gegenüber einer Edelstahl-Prüfplatte aufweist, an der die druckempfindliche Klebeschicht (12) befestigt wurde, wobei die Schälfestigkeit gemäß JIS Z 0237:2009 gemessen wird.

3. Satz von klebenden Untersuchungsmarkern nach Anspruch 1 oder 2, wobei, von einem Blickpunkt aus betrachtet, der einer Ebene zugewandt ist, in der sich das Laminat erstreckt,

> das Laminat eine elliptische Form oder eine Kapselform aufweist,
> die Hauptachse und die Nebenachse des Laminats jeweils eine Länge von 45 mm oder mehr aufweisen und

das Verhältnis der Hauptachse zur Nebenachse größer als 1 ist.

4. Satz von klebenden Untersuchungsmarkern nach einem der Ansprüche 1 bis 3, der ferner eine Schutzüberzug (13) umfasst, der auf einer Oberfläche der druckempfindlichen Klebeschicht (12) auf der dem Trägerüberzug (11) abgewandten Seite laminiert ist, wobei der Schutzüberzug (13) von der druckempfindlichen Klebeschicht (12) abziehbar ist.

5. Satz von klebenden Untersuchungsmarkern nach einem der Ansprüche 1 bis 4, wobei das Laminat eine Durchlässigkeit von 70% oder mehr für Mikrowellen mit einer Frequenz von 2 GHz aufweist.

6. Satz von klebenden Untersuchungsmarkern nach einem der Ansprüche 1 bis 5, wobei das Laminat einen Bereich umfasst, der die visuelle Erkennung des Prüfobjekts durch diesen Bereich des Laminats ermöglicht, wobei der Bereich des Laminats eine Gesamtlichtdurchlässigkeit von 30% oder mehr aufweist, wobei die Gesamtlichtdurchlässigkeit gemäß JIS K 7361-1:1997 gemessen wird.

**Revendications**

1. Ensemble de repères d'examen adhésifs comprenant :

un repère adhésif supplémentaire (10) conçu pour être utilisé conjointement avec un repère d'examen adhésif devant être fixé à une cible d'examen d'un diagnostic par imagerie utilisant des micro-ondes, et qui est conçu pour être disposé entre le repère d'examen adhésif et la cible d'examen et pour être pressé vers la cible d'examen par le repère d'examen adhésif dans un état dans lequel le repère d'examen adhésif est fixé à la cible d'examen, le repère adhésif supplémentaire comprenant un stratifié comportant un film de base ayant une épaisseur de 20 μm ou moins, et une couche d'adhésif sensible à la pression conçue pour être fixée à la cible d'examen, le film de base ayant un module d'Young de 1,0 GPa ou plus ; **caractérisé en ce que** l'ensemble de repères d'examen adhésifs comprend en outre

un repère d'examen adhésif (20A) conçu pour être fixé à la cible d'examen de telle sorte que le repère d'examen adhésif (20A) recouvre le repère adhésif supplémentaire (10) fixé à la cible d'examen,

dans lequel le repère adhésif supplémentaire (10) est conçu pour être utilisé conjointement avec le repère d'examen adhésif et est conçu pour être disposé entre le repère d'examen adhésif (20A) et la cible d'examen et pour être pressé vers la cible d'examen par le repère d'examen adhésif (20A) dans un état dans lequel le repère d'examen adhésif (20A) est fixé à la cible d'examen.

2. Ensemble de repères d'examen adhésifs selon la revendication 1, dans lequel

la couche d'adhésif sensible à la pression (12) a une épaisseur de 25 μm ou moins, le stratifié présente une résistance au pelage de 1,5 N/25 mm ou plus par rapport à une plaque d'essai en acier inoxydable à laquelle la couche d'adhésif sensible à la pression (12) a été fixée, la résistance au pelage étant mesurée conformément à la norme JIS Z 0237:2009.

3. Ensemble de repères d'examen adhésifs selon la revendication 1 ou 2 dans lequel, comme on l'observe depuis un point de vue faisant face à un plan dans lequel s'étend le stratifié,

le stratifié a une forme elliptique ou une forme de capsule, un grand axe et un petit axe du stratifié ont chacun une longueur de 45 mm ou plus, et un rapport entre le grand axe et le petit axe est supérieur à 1.

4. Ensemble de repères d'examen adhésifs selon l'une quelconque des revendications 1 à 3, comprenant en outre un film protecteur (13) stratifié sur une surface de la couche d'adhésif sensible à la pression (12) sur un côté opposé à celui faisant face au film de base (11), le film protecteur (13) pouvant être décollé de la couche d'adhésif sensible à la pression (12).

5. Ensemble de repères d'examen adhésifs selon l'une quelconque des revendications 1 à 4, dans lequel le stratifié présente une transmittance d'au moins 70 % pour les micro-ondes ayant une fréquence de 2 GHz.

6. Ensemble de repères d'examen adhésifs selon l'une quelconque des revendications 1 à 5, dans lequel le stratifié comporte une partie qui permet une reconnaissance visuelle de la cible d'examen à travers la partie du stratifié, la partie du stratifié présentant une transmittance lumineuse totale d'au moins 30 %, la transmittance lumineuse totale étant mesurée conformément à la norme JIS K 7361-1:1997.

# FIG.1

10

13 ─

11 ⎫
12 ⎬ 10A

# FIG.2

10

10E (10E1)

10E2 ─

10E2

AS

10E1

AL

# FIG.3

# FIG.4

# FIG.5

# FIG.6

# FIG.7

# FIG.8

| | MATERIAL | BASE-FILM THICKNESS (μm) | YOUNG'S MODULUS (GPa) | ADHESIVE-LAYER THICKNESS (μm) | PEEL STRENGTH (N/25mm) | MAJOR AXIS (mm) | MINOR AXIS (mm) | ITEM 1 | ITEM 2 | ITEM 3 | ITEM 4 |
|---|---|---|---|---|---|---|---|---|---|---|---|
| COMPARATIVE EXAMPLE 1 | PU | 15 | 0.005 | 15 | 3.5 | 45 | 45 | POOR | EXCELLENT | GOOD | EXCELLENT |
| COMPARATIVE EXAMPLE 2 | EVA | 15 | 0.03 | 15 | 3.7 | 45 | 45 | POOR | EXCELLENT | GOOD | EXCELLENT |
| COMPARATIVE EXAMPLE 3 | PE | 15 | 0.2 | 15 | 3.8 | 45 | 45 | POOR | EXCELLENT | GOOD | EXCELLENT |
| EXAMPLE 1 | PP | 20 | 1.6 | 15 | 2.5 | 45 | 45 | EXCELLENT | EXCELLENT | GOOD | EXCELLENT |
| COMPARATIVE EXAMPLE 4 | PET | 25 | 3.8 | 15 | 2.1 | 45 | 45 | EXCELLENT | EXCELLENT | GOOD | POOR |
| EXAMPLE 2 | PET | 12 | 3.5 | 15 | 2.8 | 45 | 45 | EXCELLENT | EXCELLENT | GOOD | EXCELLENT |
| EXAMPLE 3 | | | | 15 | 2.8 | 60 | 60 | EXCELLENT | EXCELLENT | GOOD | EXCELLENT |
| EXAMPLE 4 | | | | 15 | 2.8 | 80 | 45 | EXCELLENT | EXCELLENT | EXCELLENT | EXCELLENT |
| EXAMPLE 5 | | | | 10 | 3.0 | 80 | 45 | EXCELLENT | EXCELLENT | EXCELLENT | EXCELLENT |
| EXAMPLE 6 | | | | 15 | 1.2 | 80 | 45 | EXCELLENT | GOOD | EXCELLENT | EXCELLENT |
| EXAMPLE 7 | | | | 15 | 2.8 | 80 | 45 | EXCELLENT | EXCELLENT | EXCELLENT | EXCELLENT |
| EXAMPLE 8 | | | | 20 | 2.5 | 80 | 45 | EXCELLENT | EXCELLENT | EXCELLENT | EXCELLENT |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006052951 A
- JP 2004347660 A
- US 2011223418 A1
- WO 2012048020 A1
- WO 2017057524 A